# EUROPEAN PATENT APPLICATION

(11) **EP 4 357 357 A1**
(43) Date of publication of application: **24.04.2024**
(21) Application number: 22848285.7
(22) Date of filing: 14.07.2022
(51) Int. Cl.: C07K 14/55, C12N 15/70, C12N 15/81, C07K 19/00, C12N 15/62, C12N 15/64, A61K 38/20, A61P 35/00, A61P 37/00, A61K 47/68

(54) **HUMAN INTERLEUKIN-2 VARIANT AND USE THEREOF**

(30) Priority: 30.07.2021 CN 202110868409; 01.06.2022 CN 202210621425
(71) Applicant: Yunquan Biotechnology (Beijing) Co., Ltd., Beijing 100190 (CN)
(72) Inventor: DONG, Minghui, Shaanxi 710075 (CN); LU, Yongtao, Shaanxi 710075 (CN)
(74) Representative: Sagittarius IP
(86) International application number: PCT/CN2022/105789
(87) International publication number: WO 2023/005680

(57) **Abstract**

Provided are a human interleukin-2 variant and the use thereof. Specifically, provided is an IL-2 variant (or a derivative thereof) having eliminated or reduced affinity with a high-affinity receptor IL-2Rα/β/γ and retaining affinity with a medium-affinity receptor IL-2Rβ/γ. Also provided is a recombinant protein/fusion protein comprising an IL-2 variant and a human antibody fragment. The activity of the IL-2 variant is improved, and is close to the activity of a wild human IL-2.

## Description

The present application claims the priority of the patent application (Application No. 2021108684095) filed on July 30, 2021 and the patent application (2022106214259) filed on June 1, 2022.

### FIELD OF THE INVENTION

The present disclosure relates to a human interleukin-2 (IL-2) variant or a derivative thereof with one or more amino acid mutations. The IL-2 variant or derivative thereof has an eliminated or reduced affinity with a high-affinity receptor (IL-2Rα/β/γ) and retains the affinity with a medium-affinity receptor (IL-2Rβ/γ). The present disclosure also relates to an immunoconjugate comprising the human IL-2 variant, an encoding polynucleotide, a vector, a host cell, a pharmaceutical composition, a preparation method and a therapeutic method and use thereof.

### BACKGROUND OF THE INVENTION

Human interleukin-2 (IL-2) is also known as T cell growth factor (TCGF). The IL-2 gene is located on chromosome 4 (4q27) and includes a sequence of about 7 kb in total. IL-2 is composed of about 133 amino acids and has a molecular weight of about 15 kD. Doris Morgan et al., 1976 and 1977 have respectively found that the culture fluid of activated T cells could promote T cell proliferation. The stimulating factor in the culture fluid has been purified and identified as IL-2.

The first *in vitro* cell experiments have shown that T cells, after being activated by TCR and CD28, can secrete IL-2 and express IL-2 receptor (IL-2R) on the cell surface. The binding of IL-2 to IL-2R can cause T cell proliferation and T cell effects. IL-2 is a molecule that plays a central role in the T cell immune response. *In vivo* experiments have found that after knocking out IL-2 or its receptor, animals would develop autoimmunity. IL-2 activates not only effector cells (such as T cells and NK cells) but also regulatory T cells, thereby inhibiting excess immunity against self.

IL-2 acts through IL-2R. IL-2R consists of three subunits, IL-2Rα (i.e., CD25), IL-2Rβ (i.e., CD122), and IL-2Rγ (i.e., CD132). The three subunits can form three forms of receptors, the high-affinity receptor comprises all three subunits IL-2Rα/β/γ, the medium-affinity receptor comprises IL-2Rβ/γ, and the low-affinity receptor comprises IL-2Rα. IL-2Rβ and IL-2Rγ are required for activation of downstream signaling pathways by IL-2. When IL-2 binds to IL-2Rβ and IL-2Rγ simultaneously, the two receptor subunits form a heterodimer to phosphorylate intracellular STATS, which enters the nucleus and thereby causes the transcription and expression of corresponding genes. IL-2Rα is not required for signaling, but can promote the binding of IL-2 to IL-2Rβ and IL-2Rγ.

IL-2Rγ is expressed in all immune cells. IL-2Rβ is expressed in CD8+ T cells, NK cells and regulatory T cells, and the expression level increases after T cell activation. IL-2Rα is continuously highly expressed in regulatory T cells, and transiently expressed in activated CD8+ T cells, and then the expression level is down-regulated.

IL-2 is mainly synthesized by activated T cells, especially CD4+ helper T cells. It stimulates the proliferation and differentiation of T cells, induces the production of cytotoxic T lymphocytes (CTLs) and the differentiation of peripheral blood lymphocytes into cytotoxic cells and lymphokine-activated killer (LAK) cells, promotes the expression of cytokines and cytolytic molecules by T cells, promotes the proliferation and differentiation of B cells and immunoglobulin synthesis by B cells, and stimulates the production, proliferation and activation of natural killer (NK) cells. The ability of IL-2 to expand lymphocyte populations and improve the effector function of these cells *in vivo* leads to its anti-tumor effects, and IL-2 immunotherapy has become a treatment option for certain patients with metastatic cancer. Currently, high-dose IL-2 has been approved for the treatment of metastatic renal cell carcinoma and malignant melanoma.

The IL-2 variant disclosed in WO2009135615 has mutations at positions 20, 88 or 126. The IL-2 variant disclosed in WO2012062228 has mutations at least one of positions 38, 42, 45, 62, 68 or 88. The IL-2 variant disclosed in US8906356 has mutations at positions 91 and 126. The IL-2 variant disclosed in US9732134 has mutations at least one of positions 15, 16, 22, 84, 88 or 95. The IL-2 variant disclosed in US7803361 and US8124066 comprises a mutation R38W.

### SUMMARY OF THE INVENTION

The present disclosure relates to an IL-2 variant (or a derivative thereof) with one or more amino acid mutations, and to a conjugate thereof, and also to the use and preparation method of the IL-2 variant.

### IL-2 variants or derivatives thereof

In a first aspect, the present disclosure provides an IL-2 variant (or a derivative thereof) comprising one or more amino acid mutations compared with mature wild-type human IL-2 (SEQ ID No. 1).

The "interleukin-2" or "IL-2" refers to any naturally occurring IL-2 from any mammals, for example primates such as humans, and rodents such as mice and rats. This term encompasses both unprocessed IL-2 as well as any processed form of IL-2 derived from a cell. This term also encompasses naturally occurring IL-2 variants, for example splice variants or allelic variants.

The "wild-type human IL-2" is identical to the IL-2 variant except that it has the amino acids of the wild-type IL-2 form at each amino acid position of the IL-2 variant. The term "wild-type" is intended to encompass the inclusion of one or more naturally occurring amino acid mutations that do not affect the binding ability to IL-2 receptors.

The "mature wild-type human IL-2" refers to the mature form of wild-type human IL-2. Unprocessed human IL-2 additionally comprises an N-terminal signal peptide of about 20 amino acids, which is absent in mature IL-2 molecules. Exemplary amino acid sequences of mature wild-type human IL-2 are for example, but are not limited to, Accession No. CAA25742.1 of the INSDC database, P60568.1 of Swiss-Prot, and segment 21-153 of NP_000577.2 of Genbank. In some embodiments, the mature wild-type human IL-2 is the amino acid sequence as shown in SEQ ID No. 1. It should be noted that for the purpose of recombinant expression, additional methionine M at the N-terminus is required. However, those skilled in the art should understand that sequences with or without M at the first position all fall within the scope of "mature wild-type human IL-2" in the present application.

In some embodiments, the amino acid mutations carried out in the present application result in a shorter length of the region on IL-2 that binds to IL-2Rα (at positions of about 26 to about 47 of the amino acid) compared with mature wild-type human IL-2.

In some embodiments, amino acid mutations include replacement, deletion (or truncation), insertion (or addition), modification, and any combination thereof. To alter, for example, the binding properties of IL-2, one amino acid (for example, an amino acid with a different structure and/or chemical properties) can be used to replace another amino acid. Amino acid replacements include unnatural amino acids and 20 standard amino acids. Amino acid mutations can be generated using methods known in the art, including site-directed mutagenesis, PCR, gene synthesis, chemical modification, etc.

In some embodiments, the IL-2 variant (or a derivative thereof) according to the present disclosure has a reduced affinity with IL-2Rα, and an unchanged or increased affinity with IL-2Rβ and/or IL-2Rγ.

The "affinity" refers to the total strength of non-covalent interactions between the binding site of a molecule and a ligand thereof. Unless otherwise indicated, "affinity" herein refers to the intrinsic binding affinity that reflects the interaction between members of a binding pair, for example a receptor and a ligand, at 1:1. Affinity can usually be expressed as a dissociation constant (K_{D}), which is the ratio of the dissociation to the binding rate constants (K_{dissociation} and K_{association}, respectively). Affinity can be measured by common methods in the art, including those described herein.

In some embodiments, the IL-2 variant (or a derivative thereof) according to the present disclosure has a reduced affinity with the high-affinity receptor (IL-2Rα/β/γ), but retaining or increasing the affinity with the medium-affinity receptor (IL-2Rβ/γ).

In some embodiments, the "high-affinity IL-2 receptor" refers to a heterotrimeric form of IL-2 receptors consisting of a receptor γ subunit (also known as cytokine receptor common subunit γ, γc, or CD132), a receptor β subunit (also known as CD122 or p70), and a receptor α subunit (also known as CD25 or p55).

In some embodiments, the "medium-affinity IL-2 receptor" refers to an IL-2 receptor comprising only the γ and β subunits and no α subunit (see Olejniczak and Kasprzak, MedSci Monit14, RA179-189, 2008).

In some embodiments, the IL-2 variant (or a derivative thereof) according to the present disclosure has a reduced activation of regulatory T cells (Tregs) and/or an unaffected or increased activation of immune effector cells (for example, T cells, NK cells).

In some embodiments of the IL-2 variant (or a derivative thereof) according to the present disclosure, the mutations occur at any one or more of the following positions (continuous or discontinuous) (counting from position 1 of SEQ ID No. 1):
tyrosine (Y) at position 31, lysine (K) at position 32, asparagine (N) at position 33,
proline (P) at position 34, lysine (K) at position 35, leucine (L) at position 36,
threonine (T) at position 37, arginine (R) at position 38, methionine (M) at position 39,
leucine (L) at position 40, threonine (T) at position 41, phenylalanine (F) at position 42,
lysine (K) at position 43, mutation of phenylalanine (F) at position 44, tyrosine (Y) at position 45,
cysteine (C) at position 125.

In some embodiments of the IL-2 variant (or a derivative thereof) according to the present disclosure, the mutations occur at any one or more of positions 31 to 32 and 125 (continuous or discontinuous).

In some embodiments of the IL-2 variant (or a derivative thereof) according to the present disclosure, the mutations occur at any one or more of positions 35 to 41 and 125 (continuous or discontinuous).

In some embodiments of the IL-2 variant (or a derivative thereof) according to the present disclosure, the mutations occur at any one or more of positions 43 to 45 and 125 (continuous or discontinuous).

In some embodiments of the IL-2 variant (or a derivative thereof) according to the present disclosure, the mutations occur at any one or more of positions 31 to 45 and 125 (continuous or discontinuous).

In some embodiments of the IL-2 variant (or a derivative thereof) according to the present disclosure, the mutations occur at any one or more of positions 31 to 32, 35 to 41 and 125 (continuous or discontinuous).

In some embodiments of the IL-2 variant (or a derivative thereof) according to the present disclosure, the mutations occur at any one or more of positions 35 to 41, 43 to 45 and 125 (continuous or discontinuous).

In some embodiments of the IL-2 variant (or a derivative thereof) according to the present disclosure, the mutations occur at any one or more of positions 31 to 43 and 125 (continuous or discontinuous).

In some other embodiments, when the natural sequence documented in a public database or known literature is used as the wild-type mature IL-2, the position of amino acid mutation is counted from the amino acid A at position 1.

In some particular embodiments, provided is an IL-2 variant comprising any one of the following mutations or a combination thereof:
amino acids at positions 31-45 mutated to Gly-Gly-Asn-Pro-Met-His-Gly-Leu-Asp-Gly-Phe-Gly (SEQ ID No. 11);
amino acids at positions 31-32 mutated to Gly-Gly;
amino acids at positions 35-41 mutated to Met-His-Gly-Leu-Asp-Gly (SEQ ID No. 12);
amino acids at positions 35-43 mutated to Met-Gly-Gly-Leu-Gly-Gly (SEQ ID No. 13);
amino acids at positions 35-41 mutated to Gly-Gly-Gly-Gly-Gly-Gly (SEQ ID No. 14);
amino acids at positions 43-45 mutated to Gly;
amino acid at position 125 mutated to Ala.

In some particular embodiments, the IL-2 variant has a reduced affinity with IL-2Rα while retaining or increasing the affinity with IL-2Rβ/γ, when the IL-2 variant comprises a mutation selected from the group consisting of:
(1) mutation of YK at positions 31-32 to GG; and mutation of KLTRMLT at positions 35-41 to MHGLDG; and mutation of KFY at positions 43-45 to G; mutation of C at position 125 to A; or
(2) mutation of amino acids at positions 31-45 to GGNPMHGLDGFG, and mutation of amino acid at position 125 to A;
the mutated sequence is SEQ ID No. 2 or 15.

In some particular embodiments, the IL-2 variant has a reduced affinity with IL-2Rα while retaining or increasing the affinity with IL-2Rβ/γ, when the IL-2 variant comprises a mutation selected from the group consisting of:
mutation of KLTRMLT at positions 35-41 to MHGLDG, and mutation of amino acid at position 125 to A; the mutated sequence is SEQ ID No. 3.

In some particular embodiments, the IL-2 variant has a reduced affinity with IL-2Rα while retaining or increasing the affinity with IL-2Rβ/γ, when the IL-2 variant comprises a mutation selected from the group consisting of:
mutation of YK at positions 31-32 to GG, and mutation of amino acid at position 125 to A; the mutated sequence is SEQ ID No. 4.

In some particular embodiments, the IL-2 variant has a reduced affinity with IL-2Rα while retaining or increasing the affinity with IL-2Rβ/γ, when the IL-2 variant comprises a mutation selected from the group consisting of:
mutation of KFY at positions 43-45 to G, and mutation of amino acid at position 125 to A; the mutated sequence is SEQ ID No. 5.

In some particular embodiments, the IL-2 variant has a reduced affinity with IL-2Rα while retaining or increasing the affinity with IL-2Rβ/γ, when the IL-2 variant comprises a mutation selected from the group consisting of:
(1) mutation of YK at positions 31-32 to GG; and
(2) mutation of KLTRMI,T at positions 35-41 to MHGLDG;
(3) mutation of amino acid at position 125 to A;
the mutated sequence is SEQ ID No. 6.

In some particular embodiments, the IL-2 variant has a reduced affinity with IL-2Rα while retaining or increasing the affinity with IL-2Rβ/γ, when the IL-2 variant comprises a mutation selected from the group consisting of:
(1) mutation of KLTRMI,T at positions 35-41 to MHGLDG; and
(2) mutation of KFY at positions 43-45 to G;
(3) mutation of amino acid at position 125 to A;
the mutated sequence is SEQ ID No. 7.

In some particular embodiments, the IL-2 variant has a reduced affinity with IL-2Rα while retaining or increasing the affinity with IL-2Rβ/γ, when the IL-2 variant comprises a mutation selected from the group consisting of:
(1) mutation of YK at positions 31-32 to GG; and
(2) mutation of KFY at positions 43-45 to G;
(3) mutation of amino acid at position 125 to A;
the mutated sequence is SEQ ID No. 8.

In some particular embodiments, the IL-2 variant has a reduced affinity with IL-2Rα while retaining or increasing the affinity with IL-2Rβ/γ, when the IL-2 variant comprises a mutation selected from the group consisting of:
mutation of KLTRMI,TFK at positions 35-43 to MGGLGG, and mutation of amino acid at position 125 to A; the mutated sequence is SEQ ID No. 9.

In some particular embodiments, the IL-2 variant has a reduced affinity with IL-2Rα while retaining or increasing the affinity with IL-2Rβ/γ, when the IL-2 variant comprises a mutation selected from the group consisting of:
mutation of KLTRMLT at positions 35-41 to GGGGGG, mutation of amino acids at positions 43-45 to G, and mutation of amino acid at position 125 to A; the mutated sequence is SEQ ID No. 10.

In some particular embodiments, the amino acid sequence of the IL-2 variant (or a derivative thereof) is selected from SEQ ID Nos. 2-10.

**Table 1. Sequences of human IL-2 wild-type and variants**

| Name | Sequence | SEQ ID No |
|---|---|---|
| Mature wild type IL-2-WT | | SEQ ID No. 1 |
| IL-2-1 (31-32+35-41+43-45+125) | APTSSSTKKTQLQLEHLLLDLQMILNGINNGG NPMHGLDGFGMPKKATELKHLQCLEEELKP LEEVLNLAQSKNFHLRPRDLISNINVIVLELKG SETTFMCEYADETATIVEFLNRWITFAQSIISTL T | SEQ ID No. 2 |
| IL-2-2 (35-41+125) | APTSSSTKKTQLQLEHLLLDLQMILNGINNYK NPMHGLDGFKFYMPKKATELKHLQCLEEEL KPLEEVLNLAQSKNFHLRPRDLISNINVIVLEL KGSETTFMCEYADETATIVEFLNRWITFAQSIIS TLT | SEQ ID No. 3 |
| IL-2-3 (31-32+125) | APTSSSTKKTQLQLEHLLLDLQMILNGINNGG NPKLTRMLTFKFYMPKKATELKHLQCLEEELK PLEEVLNLAQSKNFHLRPRDLISNINVIVLELK GSETTFMCEYADETATIVEFLNRWITFAQSIIST LT | SEQ ID No. 4 |
| IL-2-4 (43-45+125) | APTSSSTKKTQLQLEHLLLDLQMILNGINNYK NPKLTRMLTFGMPKKATELKHLQCLEEELKPL EEVLNLAQSKNFHLRPRDLISNINVIVLELKGS ETTFMCEYADETATIVEFLNRWITFAQSIISTLT | SEQ ID No. 5 |
| IL-2-5 (31-32+35-41+125) | APTSSSTKKTQLQLEHLLLDLQMILNGINNGG NPMHGLDGFKFYMPKKATELKHLQCLEEEL KPLEEVLNLAQSKNFHLRPRDLISNINVIVLEL KGSETTFMCEYADETATIVEFLNRWITFAQSIIS TLT | SEQ ID No. 6 |
| IL-2-6 (35-41+43-45+125) | APTSSSTKKTQLQLEHLLLDLQMILNGINNYK NPMHGLDGFGMPKKATELKHLQCLEEELKP LEEVLNLAQSKNFHLRPRDLISNINVIVLELKG SETTFMCEYADETATIVEFLNRWITFAQSIISTL T | SEQ ID No. 7 |
| IL-2-7 (31-32+43-45+125) | APTSSSTKKTQLQLEHLLLDLQMILNGINNGG NPKLTRMLTFGMPKKATELKHLQCLEEELKPL EEVLNLAQSKNFHLRPRDLISNINVIVLELKGS ETTFMCEYADETATIVEFLNRWITFAQSIISTLT | SEQ ID No. 8 |
| IL-2-8 (35-43+125) | APTSSSTKKTQLQLEHLLLDLQMILNGINNYK NPMGGLGGFYMPKKATELKHLQCLEEELKP LEEVLNLAQSKNFHLRPRDLISNINVIVLELKG SETTFMCEYADETATIVEFLNRWITFAQSIISTL T | SEQ ID No. 9 |
| IL-2-9 (35-41+43-45+125) | APTSSSTKKTQLQLEHLLLDLQMILNGINNYK NPGGGGGGFGMPKKATELKHLQCLEEELKP LEEVLNLAQSKNFHLRPRDLISNINVIVLELKG SETTFMCEYADETATIVEFLNRWITFAQSIISTL T | SEQ ID No. 10 |
| mutation sequenceat 31-32 | GG | |
| mutation sequence at 31-45 | GGNPMHGLDGFG | SEQ ID No. 11 |
| mutation sequence at 35-41 | MHGLDG | SEQ ID No. 12 |
| mutation sequence at 35-43 | MGGLGG | SEQ ID No. 13 |
| mutation sequence at 35-41 | GGGGGG | SEQ ID No. 14 |
| mutation sequence at 43-45 | G | |
| mutation sequence at 125 | A | |
| IL-2-10 (IL-2-1+Fc) | APTSSSTKKTQLQLEHLLLDLQMILNGINNGG NPMHGLDGFGMPKKATELKHLQCLEEELKP LEEVLNLAQSKNFHLRPRDLISNINVIVLELKG SETTFMCEYADETATIVEFLNRWITFAQSIISTL TEPKSADKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSREEMTKNQVSLTCLVKGF YPSDIAVEWESNGQPENNYKTTPPVLDSDGSF FLYSKLTVDKSRWQQGNVFSCSVMHEALHNH YTQKSLSLSPGK | SEQ ID No. 15 |
| Human IgG1 Fc | EPKSADKTHTCPPCPAPELLGGPSVFLFPPKPK DTLMISRTPEVTCVVVDVSHEDPEVKFNWYV DGVEVHNAKTKPREEQYNSTYRVVSVLTVLH QDWLNGKEYKCKVSNKALPAPIEKTISKAKG QPREPQVYTLPPSREEMTKNQVSLTCLVKGFY PSDIAVEWESNGQPENNYKTTPPVLDSDGSFF LYSKLTVDKSRWQQGNVFSCSVMHEALHNH YTQKSLSLSPGK | SEQ ID No. 16 |

The naturally expressed human IL-2 has a total of 153 amino acids, with the amino acids at positions 1-20 being the signal peptide. It has a total of 133 amino acids (i.e., mature human IL-2) after cleavage of the signal peptide, corresponding to positions 1-133 of SEQ ID No. 1 in the present disclosure. For the purpose of recombinant expression, artificially produced IL-2 has an additional methionine at the first position (corresponding to the start codon AUG).

As an example, the expression "mutation of amino acids at positions 31-32 to GG" means that:
in the sequence of human IL-2 mature protein, APTSSSTKKTQLQLEHLLLDLQMILNGINN**YK**NPKLTRMLTFKFYMPKKATELK HLQCLEEELKPLEEVLNLAQSKNFHLRPRDLISNINVIVLELKGSETTFMCEYAD ETATIVEFLNRWITFCQSIISTLT (SEQ ID No. 1, 133 amino acids in total), YK at positions 31-32 are mutated to GG.

In some embodiments, the IL-2 variant is in monomeric form.

In a second aspect, the present disclosure provides a derivative of an IL-2 variant comprising the IL-2 variant of the present disclosure with a modification selected from the group consisting of: PEGylation, glycosylation, conjugation to albumin, conjugation to Fc, hydroxyethylation, de-O-glycosylation.

In some embodiments, the derivative of IL-2 variant includes the full length or a part of the IL-2 variant of the present disclosure, or a functional derivative, functional fragment, bioactive peptide, fusion protein, isoform or salt thereof further obtained based on the IL-2 variant of the present disclosure For example a fusion protein comprising the IL-2 variant, dimers or trimers or multimers of the IL-2 variant. Various modified forms of the IL-2 variant (for example, after PEGylation, glycosylation, conjugation to albumin, conjugation to Fc, hydroxyethylation, de-O-glycosylation, etc.).

In some embodiments, the derivative of IL-2 variant is PEGylated (which can be expressed as PEG-IL-2).

In some other embodiments, the PEG-IL-2 variant comprises a methoxy-PEG-aldehyde (mPEG-ALD) linker. In certain embodiments, the average molecular weight of PEG is about 5 kD to about 50 kD, particularly 5, 10, 11, 12, 13, 14, 15, 16, 17, 18, 19, 20, 21, 22, 23, 24, 25, 30, 35, 40, 45, 50 kD, or about 5 kD to about 40 kD, or about 10 kD to about 30 kD, or between about 10 kD and about 30 kD, or between about 15 kD and about 20 kD.

In certain particular embodiments, the mPEG-ALD linker comprises a PEG molecule with an average molecular weight selected from the group consisting of about 5 kDa, about 10 kDa, about 12 kDa and about 20 kDa. In some embodiments, the aldehyde group of mPEG-ALD can be acetaldehyde, propionaldehyde, or butyraldehyde, etc.

In one embodiment, the derivative of IL-2 variant has an extended serum half-life compared with the mature wild-type human IL-2.

In some embodiments, the derivative of IL-2 variant is an IL-2 variant conjugated to an antibody or an antigen-binding fragment thereof. In some embodiments, the derivative of IL-2 variant is an IL-2 variant conjugated to the Fc fragment of a human antibody. In particular embodiments, the derivative of IL-2 variant is an IL-2 variant conjugated to the Fc fragment of a human antibody at its C-terminus (i.e., the IL-2 variant according to the present application is conjugated to the N-terminus of the Fc fragment of a human antibody).

In some cases, the derivative of IL-2 variant is in a dimer form, either a homodimer or a heterodimer. As an example, a modification is introduced into the Fc fragment to promote heterodimerization. In some embodiments, the Fc fragment comprises a knob-into-hole (KIH) structure, which involves introducing a knob structure at the interface of the first Fc fragment as well as a hole structure at the interface of the second Fc fragment, and vice versa. This allows the knob structure to be localized in the hole structure, promotes the formation of heterodimers, and inhibits the formation of homodimers. The knob structure is constructed by replacing the small amino acid side chain from the interface of the first Fc fragment with a larger side chain, such as tyrosine or tryptophan, whereas the pore structure is created by replacing the large amino acid side chain with a smaller amino acid side chain, such as alanine or threonine, at the interface of the second Fc fragment.

### Conjugates

In a third aspect, the present disclosure provides a conjugate comprising a first component and a second component, wherein the first component is directly conjugated to (or indirectly conjugated through a linker to) the second component; the first component is the IL-2 variant (or a derivative thereof) of the present disclosure; the second component is not an IL-2 or an IL-2 variant (or a derivative thereof).

In some embodiments, the IL-2 variant (or a derivative thereof) is connected to at least one second component.

In some embodiments, the IL-2 variant (or a derivative thereof) and the second component forms a fusion protein through peptide bonds.

In some particular embodiments, the IL-2 variant (or a derivative thereof) is conjugated to (or indirectly conjugated through a linker to) the carboxyl terminus of the second component.

In some particular embodiments, the IL-2 variant (or a derivative thereof) is conjugated to (or indirectly conjugated through a linker to) the amino terminus of the second component.

In some embodiments, the second component is an antigen-binding component. The antigen-binding component refers to a polypeptide molecule that specifically binds an epitope.

In some embodiments, the antigen-binding component can direct a moiety connected to it (for example, the IL-2 of the present disclosure, a variant or a derivative thereof) to a target site (for example, to a tumor cell or tumor stroma with antigen determinants). The antigen-binding component can be an antibody or an antigen-binding fragment.

The antibody is used in the broadest sense herein and encompasses various antibody structures as long as they exhibit antigen-binding activity. The antibody includes, but is not limited to, monoclonal antibody, polyclonal antibody, multispecific antibody (such as bispecific antibody) and antigen-binding fragment. The antibody can include murine antibody, human antibody, humanized antibody, chimeric antibody and camel antibody.

In some particular embodiments, the antibody or antigen-binding fragment thereof is selected from the group consisting of a polypeptide complex comprising antibody heavy chain variable region and antibody light chain variable region, Fab, Fv, sFv, F(ab')2, linear antibody, single-chain antibody, scFv, sdAb, sdFv, nanobody, peptibody, domain antibody and multispecific antibody (bispecific antibody, diabody, triabody and tetrabody, tandem di-scFv, tandem tri-scFv).

In some embodiments, when the IL-2 variant (or a derivative thereof) is connected to more than one antigen-binding component, each antigen-binding component can be independently selected from an antibody and an antigen-binding fragment. For example, the first antigen-binding component can be a Fab molecule, and the second antigen-binding component can be a scFv molecule. For example, the first antigen-binding component can be a scFv molecule, while the second antigen-binding component is also a scFv molecule. In some embodiments, the first and second antigen-binding components are independently directed against different antigens or against the same antigen. In some embodiments, the antibody or antigen-binding fragment thereof targets a tumor antigen.

In some embodiments, the tumor antigen is for example, but not limited to, MAGE family (for example, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2, MAGE-B2, MAGE-Xp3, MAGE-B3, MAGE-Xp4, MAGE-B4, MAGE-C1, MAGE-C2, MAGE-C3, MAGE-C4, MAGE-C5), GAGE family (for example, GAGE-1, GAGE-2, GAGE-3, GAGE-4, GAGE-5, GAGE-6, GAGE-7, GAGE-8, GAGE-9), MART-1/Melan-A, gp75, gp100, DPPIV, ADAbp, cyclophilin, CEA, CAP-1, CAP-2, etv6, aml1, PSA (PSA-1, PSA-2 and PSA-3), PSMA, T cell receptor, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, PRAME, NY-ESO-1, cdc27, SSX-1, SSX-2, SSX-1, SSX-4, SSX-5, SCP-1, CT-7.

### Pharmaceutical compositions

In a fourth aspect, the present disclosure provides a pharmaceutical composition containing the IL-2 variant (or a derivative thereof) or the conjugate according to the present disclosure, and optionally a pharmaceutically acceptable diluent, carrier or excipient. The pharmaceutical composition can be a lyophilized preparation or an injectable solution.

In some particular embodiments, the unit dose of the pharmaceutical composition can contain 0.01 wt% to 99 wt% of the IL-2 variant (or a derivative thereof) or the conjugate; or, the amount of the IL-2 variant (or a derivative thereof) or the conjugate contained in the unit dose of the pharmaceutical composition is 0.1 to 2000 mg (for example, 1-1000 mg; 1, 5, 10, 15, 20, 25, 30, 35, 40, 45, 50, 55, 60, 65, 70, 75, 80, 85, 90, 5, 100, 110, 120, 130, 140, 150, 160, 170, 180, 190, 200, 250, 300, 350, 400, 450, 500, 550, 600, 650, 700, 750, 800, 850, 900, 950, 1000 mg).

### Polynucleotides

In a fifth aspect, the present disclosure provides a polynucleotide encoding the IL-2 variant (or a derivative thereof) of the present disclosure.

Those skilled in the art understand that the nucleotide sequences encoding the same amino acid sequence can be different. Due to codon degeneracy and codon bias in different hosts, different nucleotide sequences can encode the same amino acid sequence, and these sequences all fall within the scope of the present disclosure.

### Expression vectors

In a sixth aspect, the present disclosure provides an expression vector containing the polynucleotide according to the present disclosure. The expression vector can be an eukaryotic expression vector, a prokaryotic expression vector or a shuttle expression vector.

### Host cells

In a seventh aspect, the present disclosure provides a host cell that comprises the expression vector according to the present disclosure, or expresses the IL-2 variant according to the present disclosure, or expresses the conjugate according to the present disclosure.

In some embodiments, the host cell comprises (for example it has been transformed or transfected with) an expression vector comprising the polynucleotide according to the present disclosure.

In some embodiments, the host cell is a prokaryotic or eukaryotic cell.

In some embodiments, the host cell is a bacterial, yeast or mammalian cell, in particular *Pichia pastoris* or *Saccharomyces cerevisiae.*

In some particular embodiments, the host cell is a prokaryotic microorganism, such as *E*. *coli.*

In some particular embodiments, the host cell is a eukaryotic cell.

In some embodiments, host cells such as plant and insect cells that express glycosylated peptides are used. Vertebrate cells can also be used as the host cell, for example, mammalian cell lines in suspension culture, monkey kidney CV1 cell line (COS-7), human embryonic kidney cell line (293 or 293T cells), baby hamster kidney cells (BHK), mouse sertoli cells (TM4 cells), monkey kidney cells (CV1), VERO-76, human cervical cancer cells (HELA), canine kidney cells (MDCK), buffalo rat liver cells (BRL3A), human lung cells (W138), human liver cells (Hep G2), mouse breast tumor cells (MMT060562), MRC5 cells, FS4 cells, CHO cells, myeloma cell lines (such as YO, NS0, P3X63 and Sp2/0).

### Uses and treatment methods

In an eighth aspect, the present disclosure provides use of the IL-2 variant (or a derivative thereof), the conjugate and the pharmaceutical composition according to the present disclosure in the manufacture of a medicament.

In some embodiments, provide is use of the IL-2 variant (or a derivative thereof) in treating proliferative diseases and immune diseases, in modulating T cell-mediated immune responses, and in stimulating the individual's immune system.

In some embodiments, the proliferative disease can be a tumor or cancer (for example, a metastatic tumor or cancer) or can be a solid tumor.

In some embodiments, the IL-2 variant (or a derivative thereof), the conjugate, or the pharmaceutical composition of the present disclosure can be used to treat and stimulate the host's immune system (in particular, to enhance the cellular immune response).

In some embodiments, enhancing the cellular immune response can include, but is not limited to, improving T cell function, improving B cell function, restoring lymphocyte function, increasing the expression of IL-2 receptor, increasing T cell responsiveness, increasing the activity of natural killer cells or lymphokine-activated killer (LAK) cells.

In some embodiments, the IL-2 variant (or a derivative thereof), the conjugate, or the pharmaceutical composition of the present disclosure is used for treating a proliferative condition, such as cancer. Non-limiting examples of cancer include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell cancer, bone cancer and kidney cancer.

In some embodiments, the IL-2 variant (or a derivative thereof), the conjugate, or the pharmaceutical composition of the present disclosure is used for treating a neoplasm located in the abdomen, bone, breast, digestive system, liver, pancreas, peritoneum, endocrine gland (adrenal gland, parathyroid gland, pituitary, testis, ovary, thymus, thyroid), eye, head and neck, nervous system (central and peripheral), lymphatic system, pelvis, skin, soft tissue, spleen, chest and genitourinary system.

In some embodiments, the IL-2 variant (or a derivative thereof), the conjugate, or the pharmaceutical composition of the present disclosure is used for treating a precancerous condition or cancer metastasis. The cancer is selected from the group consisting of renal cell carcinoma, skin cancer, lung cancer, colorectal cancer, breast cancer, brain cancer and head and neck cancer.

In some embodiments, the IL-2 variant (or a derivative thereof), the conjugate, or the pharmaceutical composition of the present disclosure is used for treating hypergammaglobulinemia, lymphoproliferative condition, paraproteinemias, purpura, sarcoidosis, Sezary syndrome, Waldenstron's macroglobulinemia, Gaucher's disease, histiocytosis.

In some embodiments, the IL-2 variant (or a derivative thereof), the conjugate, or the pharmaceutical composition of the present disclosure is used for treating autoimmune disease, transplant rejection, post-traumatic immune response and infectious disease.

In some embodiments, the autoimmune disease can be selected from the group consisting of type I diabetes mellitus, rheumatoid arthritis, multiple sclerosis, chronic gastritis, Crohn's disease, Basedow disease, Bechterew disease, psoriasis, myasthenia gravis, autoimmune hepatitis, APECED, Chrug-Strauss syndrome, ulcerative colitis, glomerulonephritis, Guillain-Barré syndrome, Hashimoto thyroiditis, lichen sclerosus, systemic lupus erythematodes, PANDAS, rheumatic fever, sarcoidosis, Sjögren syndrome, Stiff-Man syndrome, scleroderma, Wegener's granulomatosis, vitiligo, autoimmune enteropathy, Goodpasture syndrome, dermatomyositis, polymyositis, autoimmune allergy, asthma.

In some embodiments, the IL-2 variant (or a derivative thereof) can be used in combination with an immunosuppressant.

In some embodiments, the immunosuppressant is selected from the group consisting of glucocorticoid, azathioprine, cyclosporin A; mycophenolatemofetil, tacrolimus, anti-CD3 antibody, anti-CD25 antibody, anti-TNF-α antibody, methotrexate, cyclosporin, sirolimus, everolimus, fingolimod and cyclophosphamide.

In some embodiments, a therapeutically effective amount of the IL-2 variant (or a derivative thereof), the conjugate or the pharmaceutical composition of the present disclosure is administered to a subject.

In some embodiments, the subject in need, such as a patient or an individual, is usually a mammal, such as a human.

In some embodiments, the IL-2 variant (or a derivative thereof), the conjugate or the pharmaceutical composition of the present disclosure is administered to a subject at least twice a day, at least once a day, at least once every 48 hours, at least once every 72 hours, at least once a week, at least once every 2 weeks, at least once a month, at least once every 2 months, or at least once every 3 months.

In some embodiments, tthe IL-2 variant (or a derivative thereof), the conjugate or the pharmaceutical composition of the present disclosure is administered via any route, for example, administered by parenteral injection (for example, subcutaneous or intravenous injection).

### DESCRIPTION OF THE DRAWINGS

Figure 1. The curves of interactions between wild-type human IL-2, IL-2 variants or derivatives thereof and IL-2Rα receptors.
Figure 2. The curves of interactions between wild-type human IL-2, IL-2 variants or derivatives thereof and IL-2Rβ/γ receptors.

### DETAILED DESCRIPTION OF THE INVENTION

The following examples are incorporated for further description of the present disclosure, but these examples do not limit the scope of the present disclosure.

### Example 1. Recombinant expression and preparation of wild-type human IL-2 and IL-2 variants or derivatives thereof

Synthesis, expression and purification of nucleic acid sequences of IL-2 variants.
1. In this example, wild-type human IL-2 and IL-2 variants (IL-2-1 to IL-2-9) were expressed separately, and purified and prepared using the HPC4 tag carried at the C-terminus of the molecules. IL-2-10 was an IL-2-1 variant with C-terminal addition of human IgG1 Fc sequence, and it was then purified and prepared.

The whole gene was synthesized, and the coding sequence of each protein was subcloned into the expression vector pcDNA3.1 by double enzyme digestion. The final expression vectors were confirmed for their accuracy by enzyme digestion and sequencing, and finally transfected into DH5α clone strain. The plasmids suitable for transfection were extracted by plasmid extraction kit, transfected into mammalian HEK293 cells using transfection reagents for transient expression, and each protein was purified by affinity chromatography.

### 2. Plasmid transformation of DH5α

80-100 ng of the expression plasmids containing the coding sequences were pipetted into previously prepared DH5α competent cells. The competent cells were transformed by heat shock after addition of plasmids.

The transformed competent cells were added into LB liquid medium, placed in a shaker at 37°C, and incubated while shaking at 200 rpm for about 30 min. The cultured competent cells were taken out from the shaker, and a part of the suspension was pipetted and spread on a plate containing ampicillin, which was placed in an incubator and incubated at 37°C overnight.

Single clones from the fresh culture plate were picked and incubated in 2-5 mL of LB medium at 37°C, 200 rpm for 8 h. The culture was inoculated into 200 mL of LB medium at a ratio of 1/500 and incubated at 37°C, 200 rpm for 16 h. The cultured bacterial suspension was collected and centrifuged to remove the supernatant.

### 3. Thawing and passage of HEK293 cells

A water bath was set to 37°C to pre-warm the medium at 37°C.

HEK293 cells were taken out from a liquid nitrogen tank, immediately put into the water bath at 37°C and shaken gently for quick thawing (in about 1 min).

The outer wall of tubes was sterilized with 75% ethanol, and the tubes were placed in a biosafety cabinet. The cells were transferred to a 15 mL centrifuge tube containing 10 mL of medium, and centrifuged at 800 rpm for 5 min. After centrifugation, the supernatant was removed, and the cells were resuspended in a little volume of fresh medium, and then transferred to a culture flask. Fresh medium was added and the flask was shaken gently for uniform dispersion of cells, and cell samples were collected for counting and viability detection. The density was controlled at 3×10⁵ to 4×10⁵ cells/mL with a viability of > 95%.

The cells were placed in an incubator at 37°C, 110 rpm with 5% CO₂. After 2-3 days of cell culture to reach a density of 2.0×10⁶ cells/mL, the cells were passaged by supplementing with fresh medium. The cells were monitored for density and viability.

### 4. Plasmid transfection of HEK293 cells

One day before transfection, HEK293 cells were cultured in suspension in 1 L with an inoculation density of 1×10⁶ cells/mL, placed in an incubator and cultured at 37°C, 110 rpm with 5% CO₂. On the day of transfection, the cell density was controlled at 1×10⁶ cells/mL to 1.5×10⁶ cells/mL.

Mixture of DNA-transfection reagents: DNA and transfection reagents were added to a transfection buffer, mixed well and incubated at 37°C. The mixture of DNA-transfection reagents was added to the cells to be transfected, placed in an incubator and incubated at 37°C, 110 rpm with 5% CO₂. About 4-6 days after transfection, the cell culture was taken out from the incubator and centrifuged to collect the supernatant and the cells.

### 5. Protein purification

The cell culture medium after 5 days of culture was collected and centrifuged, and then the supernatant was collected and filtered through a 0.22 µm filter. The sample was dialyzed at 4°C with a buffer of 1× PBS, pH 7.4. After dialysis, the sample was subjected to affinity purification using medium conjugated with HPC4 antibodies, and then to gel filtration chromatography with superdex200 for further purification to obtain the target protein with high purity.

### Example 2. Determination of affinity of wild-type human IL-2 and IL-2 variants or derivatives thereof with IL-2Rα

The binding properties of wild-type human IL-2 and IL-2 variants or derivatives thereof (IL-2-1 to IL-2-10) obtained in Example 1 for IL-2Rα were detected by an Octet platform. The Octet platform detected and analyzed the interactions between biomolecules based on Bio-Layer Interferometry (BLI).
1. Experimental instruments, reagents and consumables
   Octet^{®} Octet RED96e system (Pall Fortebio Corp, Menlo Park, CA);
   analysis buffer: SD buffer (PBS (pH 7.4) + 0.01% BSA + 0.02% Tween20);
   acetic acid buffer (10 mM, pH 4.0, 5.0, 6.0);
   AR2G (Fortebio, catalog no. 18-5092);
   96-well plates (Greiner Bio-One part no. 655209).
2. The experiment was performed with reference to the experimental design below. The whole experiment was completed under the conditions of 30°C, 1000 rpm, 220 µl/well. The proteins used in the experiment were IL2-Rα-his and IL-2 variants or derivatives thereof obtained by transient expression in HEK293 and affinity purification.

The analyte sample was IL-2Rα, and the dilution buffer was SD buffer;
the analyte concentrations were 500 nM, 240 nM, 120 nM, 60 nM, 30 nM, 15 nM (and an additional concentration of 1000 nM for some variants).

### 3. Experimental data and processing

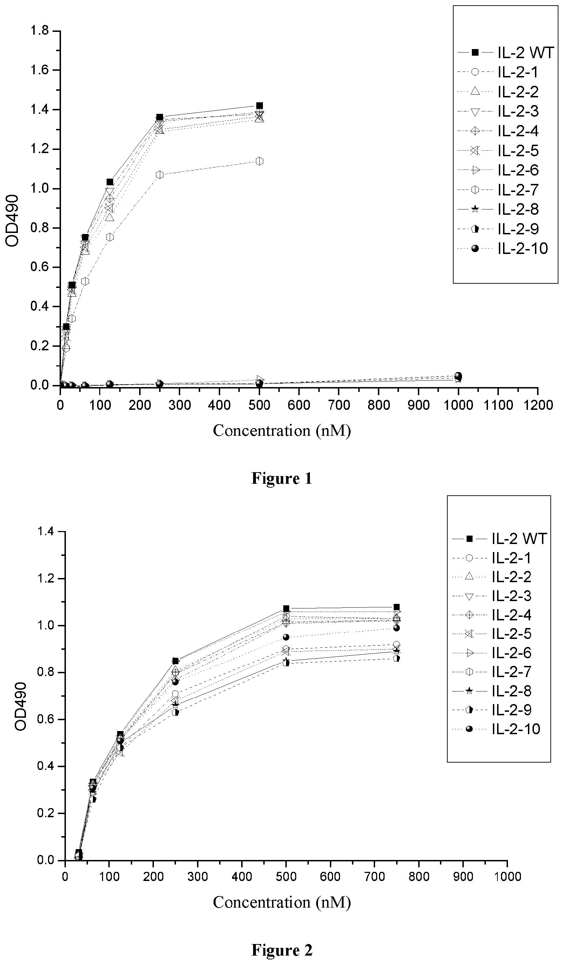

Experimental data were processed using Data Analysis software 9.0. A 1:1 model was selected for fitting, i.e., 1 wild-type human IL-2 or IL-2 variant (specifically IL-2-1 to IL-2-10) bound to 1 IL-2Rα. The method of global fitting was applied, i.e., the 6 concentrations were analyzed as a group. The fitting results were shown in Figure 1.

The data obtained in the experiment were fitted against a 1:1 binding model to obtain the affinity Kd value of wild-type human IL-2 or IL2 variants or derivatives with the receptor IL-2Rα.

Binding data of wild-type human IL-2 and IL-2 variants or derivatives thereof with IL-2Rα were shown in Figure 1 and Table 2.

The results showed that IL-2-1, IL-2-6, IL-2-8, IL-2-9 and IL-2-10 did not bind to IL-2Rα, and the interaction between IL-2-2, IL-2-3, IL-2-4, IL-2-5, IL-2-7 and IL-2Rα was reduced to a certain extent or retained.

This suggested that:
1) mutation of amino acids at positions 31-32 to GG did not significantly affect the binding of IL-2 to IL-2Rα;
2) mutation of amino acids at positions 43-45 to G had a certain effect on the binding of IL-2 to IL-2Rα;
3) mutation of KLTRMLT at positions 35-41 to MHGLDG did not significantly affect the binding of IL-2 to IL-2Rα;
4) the combination of the above-mentioned three mutations greatly changed the protein structure, thereby greatly reducing the binding of IL-2 to IL-2Rα, and no binding of the two was observed under experimental conditions.

**Table 2. Affinity of IL-2 wild-type and variants or derivatives thereof with IL-2 receptor α**

| Name | Kd (nM) |
|---|---|
| IL-2WT | 0.932 |
| IL-2-1 | N.A. |
| IL-2-2 | 0.789 |
| IL-2-3 | 0.656 |
| IL-2-4 | 0.799 |
| IL-2-5 | 0.904 |
| IL-2-6 | N.A. |
| IL-2-7 | 3.25 |
| IL-2-8 | N.A. |
| IL-2-9 | N.A. |
| IL-2-10 | N.A. |
| N.A., not available. Kd could not be obtained by fitting the data within the concentration range used in the experiment due to the low binding of these mutations to IL-2Rα. | |

### Example 3. Determination of affinity of wild-type human IL-2 and IL-2 variants or derivatives thereof with IL-2Rβ/γ

The affinity of wild-type human IL-2 and variants or derivatives thereof in Example 1 with IL-2Rβ/γ was detected by experiments.
1. Experimental instruments, reagents and consumables
   Octet^{®} Octet RED96e system (Pall Fortebio Corp, Menlo Park, CA);
   analytical buffer: SD buffer (PBS (pH 7.4) + 0.01% BSA + 0.02% Tween20);
   acetic acid buffer (10 mM, pH 4.0, 5.0, 6.0);
   AR2G (Fortebio, catalog no. 18-5092);
   96-well plates (Greiner Bio-One part no. 655209).
   IL-2Rβ and IL-2Rγ subunits were cloned and fused to Fc hole and Fc knob respectively for the preparation of IL-2Rβ/γ-Fc heterodimers. IL-2Rβ-Fc-hole and IL-2Ry-Fc-knob were simutaneously transfected into HEK293 cells. The heterodimers were purified with Protein A and molecular sieve Superdex 200.
2. The whole experiment was completed under the conditions of 30°C, 1000 rpm, 220 µl/well.

The analyte sample was IL-2Rβ/γ heterodimers and the dilution buffer was SD buffer. The analyte concentrations were 750 nM, 500 nM, 250 nM, 125 nM, 62.5 nM, 31.3 nM.

### 3. Experimental data and processing

Experimental data were processed using Data Analysis software 9.0. A 1:1 model was selected for fitting, i.e., 1 wild-type human IL-2 or IL-2 variant or derivative thereof (IL-2-1 to IL-2-10) bound to 1 IL-2Rβ/γ. The method of global fitting was applied, i.e., the 6 concentrations were analyzed as a group.

The data obtained in the experiment were fitted against a 1:1 binding model to obtain the affinity Kd value of wild-type human IL-2 or IL2 variants or derivatives thereof with IL-2Rβ/γ, and the binding data were shown in Figure 2 and Table 3.

The results showed that the interaction between IL-2 variants or derivatives thereof (IL-2-1 to IL-2-10) and IL-2Rβ/γ remained unchanged.

**Table 3. Affinity of IL-2 wild-type and variants or derivatives thereof with IL-2Rβ/γ**

| Name | Kd (nM) |
|---|---|
| IL-2WT | 4.9 |
| IL-2-1 | 17.9 |
| IL-2-2 | 4.87 |
| IL-2-3 | 3.74 |
| IL-2-4 | 4.11 |
| IL-2-5 | 5.24 |
| IL-2-6 | 19.56 |
| IL-2-7 | 6.48 |
| IL-2-8 | 21.34 |
| IL-2-9 | 27.2 |
| IL-2-10 | 9.83 |

### Example 4. CTLL-2 cell proliferative activity assay for wild-type IL-2, IL-2 variants or derivatives thereof

The biological activity of IL-2 was detected according to the proliferation rate of the dependent cell strain CTLL-2 under different IL-2 concentrations.

Cells were cultured in RPMI 1640 medium containing 10% fetal bovine serum and 1% of the two antibiotics (Penicillin-Streptomycin solution) in an incubator at 37°C with 5% CO₂. Cells in the logarithmic growth phase were trypsinized, counted under a microscope, and then prepared as cell suspensions of 1×10⁴ to 5×10⁴ cells/ml. 100 µl of suspension was pipetted to three identical wells in a 96-well culture plate as triplicates per plate for each type of cells, at 1×10³ to 5×10³ cells/well, with 100 µl of medium as the blank control, and the plates were incubated at 37°C overnight.

Groups with the following different concentrations were set: blank, 0.001 nM, 0.01 nM, 0.1 nM, 1 nM, 10 nM.

At 0 h and 24 h, Cell Counting Kit-8 (CCK-8) and serum-free minimum essential medium were mixed at a volume ratio of 1: 10, added to the test wells at 100 µL per well, and incubated in an incubator at 37°C with 5% CO₂ for 1 h.

The absorbance at a wavelength of 490 nm was determined with a microplate reader. The values of each plate were recorded.

The results were as shown in Table 4. The IL-2 variants or derivatives thereof all had CTLL-2 cell proliferative activity, indicating that mutations did not significantly affect the signaling function of the IL-2Rβ/γ receptor subunit complex. The proliferative activity of IL-2-1, IL-2-6, IL-2-8 and IL-2-9 variants on CTLL-2 cells was significantly lower than that of the positive control, because these IL-2 variants did not bind to the IL-2Rα receptor, while the positive control bound to the IL-2Rα receptor, thereby enhancing the binding to IL-2Rβ/γ, such that the proliferative activity on CTLL-2 cells was higher.

IL-2-10 was a recombinant protein formed by connecting the IL-2-1 variant at its C-terminus to the N-terminus of the Fc fragment (defined as a derivative of an IL-2 variant in the present application). Because it had a sufficient molecular weight to enhance the binding to IL-2Rβ/γ, it could maintain a proliferative activity on CTLL-2 cells similar to that of wild-type human IL-2. The data were shown in Table 4.

**Table 4**

| Name | Relative activity |
|---|---|
| IL-2WT | 100% |
| IL-2-1 | 13% |
| IL-2-2 | 64% |
| IL-2-3 | 100% |
| IL-2-4 | 100% |
| IL-2-5 | 90% |
| IL-2-6 | 13% |
| IL-2-7 | 58% |
| IL-2-8 | 8% |
| IL-2-9 | 8% |
| IL-2-10 | 74.9% |

## Claims

1. A human interleukin-2 (IL-2) variant comprising an amino acid mutation compared with mature wild-type human IL-2, the mutation is at least one of replacement, deletion and addition; preferably, the mature wild-type human IL-2 is as shown in SEQ ID No. 1;
wherein, the mutation is selected from the group consisting of:
mutation of the amino acid residues at positions 31 to 45 of the mature wild-type human IL-2 to the sequence as shown in SEQ ID No. 11;
mutation of the amino acid residues at positions 35 to 41 of the mature wild-type human IL-2 to the sequence as shown in SEQ ID No. 12 or 14;
mutation of the amino acid residues at positions 35 to 43 of the mature wild-type human IL-2 to the sequence as shown in SEQ ID No. 13;
mutation of the amino acid residues at positions 31 to 32 of the mature wild-type human IL-2 to GG;
mutation of the amino acid residues at positions 43 to 45 of the mature wild-type human IL-2 to G;
mutation of the amino acid residue at position 125 of the mature wild-type human IL-2 to A;
optionally, addition of M at the N-terminus of the mature wild-type human IL-2.

2. The IL-2 variant according to claim 1, wherein the IL-2 variant is as shown in a sequence selected from the group consisting of:
SEQ ID Nos. 2 to 10, SEQ ID No. 15;
SEQ ID Nos. 2 to 10 with addition of M at the N-terminus, and SEQ ID No. 15 with addition of M at the N-terminus.

3. The IL-2 variant according to any one of claims 1 to 2, wherein:
the IL-2 variant has a reduced affinity with IL-2Rα; and/or
the IL-2 variant has an unchanged or increased affinity with IL-2Rβ; and/or
the IL-2 variant has an unchanged or increased affinity with IL-2Rγ;
preferably, the IL-2 variant has a reduced affinity with IL-2Rα/β/γ, and the affinity of the IL-2 variant with IL-2Rβ/γ is retained or increased.

4. A derivative of an IL-2 variant comprising the IL-2 variant according to any one of claims 1 to 3 with a modification selected from the group consisting of:
PEGylation, glycosylation, conjugation to albumin, conjugation to Fc, hydroxyethylation, de-O-glycosylation;
preferably, the conjugation to Fc refers to conjugation of human IgG1 Fc to the C-terminus of the IL-2 variant according to any one of claims 1 to 3;
the PEGylation refers to connection of PEG at the N-terminus of the IL-2 variant;
peferably, the PEG has a molecular weight of 5 kD to 80 kD;
more preferably, the PEG has a molecular weight of 10 kD to 20 kD.

5. A conjugate comprising:
a first component, and
a second component;
wherein: the first component is directly conjugated to, or indirectly conjugated through a linker to, the second component;
the first component is the IL-2 variant according to any one of claims 1 to 3, or the derivative of an IL-2 variant according to claim 4;
preferably, the second component is an antibody or antigen-binding fragment thereof;
more preferably, the antibody or antigen-binding fragment thereof targets a tumor antigen.

6. A pharmaceutical composition containing:
the IL-2 variant according to any one of claims 1 to 3, or the derivative of an IL-2 variant according to claim 4, or the conjugate according to claim 5;
a pharmaceutically acceptable diluent, carrier or excipient.

7. A nucleic acid molecule encoding the IL-2 variant according to any one of claims 1 to 3, the derivative of an IL-2 variant according to claim 4, or the conjugate according to claim 5.

8. An expression vector comprising the nucleic acid molecule according to claim 7.

9. A host cell comprising or expressing any one of the following:
comprising the expression vector according to claim 8,
expressing the IL-2 variant according to any one of claims 1 to 3,
expressing the derivative of an IL-2 variant according to claim 4,
expressing the conjugate according to claim 5;
preferably, the host cell is a prokaryotic or eukaryotic cell;
preferably, the host cell is selected from the group consisting of: bacterial cell, yeast cell, mammalian cell;
most preferably, the host cell is a *Saccharomyces cerevisiae* cell or an *Escherichia coli* cell.

10. Use of any one selected from the following in preparing a medicament:
the IL-2 variant according to any one of claims 1 to 3, the derivative of an IL-2 variant according to claim 4, the conjugate according to claim 5;
the medicament is for preventing or treating a disease selected from the group consisting of: a proliferative disease, a metastasis of proliferative disease, an immune disease;
preferably, the proliferative disease is a tumor or cancer;
preferably, the immune disease is selected from the group consisting of diabetes mellitus, rheumatoid arthritis, multiple sclerosis, systemic lupus erythematosus, eczema, asthma, autoimmune disease and autoimmune reaction after organ transplantation;
preferably, the tumor or cancer is selected from the group consisting of epithelial cell carcinoma, endothelial cell carcinoma, squamous cell carcinoma, carcinoma caused by papillomavirus, adenocarcinoma, melanoma, sarcoma, teratoma, lung cancer, metastatic lung cancer, lymphoma and metastatic renal cell carcinoma.
